# EUROPEAN PATENT APPLICATION

(11) **EP 4 699 521 A1**
(43) Date of publication of application: **25.02.2026**
(21) Application number: 25197661.9
(22) Date of filing: 22.08.2025
(51) Int. Cl.: A61B 5/00, A61B 5/0205

(54) **METHOD TO PRIORITIZE AND REASSEMBLE HEMODYNAMIC DATA FROM MULTIPLE TECHNOLOGY SOURCES**

(30) Priority: 23.08.2024 US 202463686309 P
(71) Applicant: Becton, Dickinson and Company, Franklin Lakes, NJ 07417 (US)
(72) Inventor: NEMES, Kristen Samantha, Irvine, CA 92614 (US); ZHAO, Winfield, Irvine, CA 92614 (US); LESHER, Caroline, Irvine, CA 92614 (US); KALE, Rohit Pandurang, Irvine, CA 92614 (US); COE, Andrew, Irvine, CA 92614 (US)
(74) Representative: Venner, Julia Ann

(57) **Abstract**

A method for monitoring a hemodynamic parameter of a patient includes sensing the hemodynamic parameter with a first sensor and communicating a first dataset of the hemodynamic parameter to a hemodynamic monitor throughout a monitoring period. A second sensor senses the hemodynamic parameter of the patient and communicates a second dataset of the hemodynamic parameter to the hemodynamic monitor throughout the monitoring period. The hemodynamic monitor saves the first dataset and the second dataset to a memory of the hemodynamic monitor. In a first step, a processor populates a data sequence with all of the values of the first dataset relative to time of the monitoring period. In a second step, the processor populates any segments of time missing a hemodynamic parameter value in the data sequence with a hemodynamic parameter value from a corresponding time segment in the second dataset after completion of the first step.

## Description

### CROSS-REFERENCE TO RELATED APPLICATION(S)

This application claims the benefit of U.S. Provisional Application No. 63/686,309, filed August 23, 2024, and entitled "METHOD TO PRIORITIZE AND REASSEMBLE HEMODYNAMIC DATA FROM MULTIPLE TECHNOLOGY SOURCES," the disclosure of which is hereby incorporated by reference in its entirety.

### BACKGROUND

The present disclosure relates generally to hemodynamic monitoring.

Hemodynamic monitoring of patients helps physicians make better clinical decisions for patients by detecting conditions and symptoms related to hemodynamic instability in the patients, such as (but not limited to) hypoperfusion, hypotension, hypertension, and arrhythmia. A sensor is placed on (non-invasively) the patient or in (invasively) the patient. The sensor is connected to a monitor that collects and displays sensed data of the patient communicated by the sensor. The physician can consult the collected data live or retroactively to determine hemodynamic parameters of the patient and the frequency of hemodynamic instability. Based on the sensed data of the patient, the physician can determine a course of action for treating the patient. The physician can also use the sensed data from the patient to make better clinical decisions for treating future patients.

### SUMMARY

In one aspect of the disclosure, a method for monitoring of a hemodynamic parameter of a patient is disclosed. The method includes sensing the hemodynamic parameter of the patient with a first sensor. The first sensor communicates a first dataset of the hemodynamic parameter to a hemodynamic monitor throughout a monitoring period of the patient. The hemodynamic monitor saves the first dataset to a memory of the hemodynamic monitor. The method also includes sensing the hemodynamic parameter of the patient with a second sensor. The second sensor communicates a second dataset of the hemodynamic parameter to the hemodynamic monitor throughout the monitoring period. The hemodynamic monitor saves the second dataset to the memory of the hemodynamic monitor. A processor assigns a first priority to the first dataset. The processor assigns a second priority to the second dataset. In a first step, the processor populates a data sequence with all of the values of the first dataset relative to time of the monitoring period. In a second step, the processor populates any segments of time missing a hemodynamic parameter value in the data sequence with a hemodynamic parameter value from a corresponding time segment in the second dataset after completion of the first step. The processor outputs the data sequence to a display.

In another aspect of the disclosure, a method for monitoring of a hemodynamic parameter of a patient is disclosed. The method includes receiving, at a hemodynamic monitor, a first dataset of a hemodynamic parameter of the patient from a first sensor throughout a monitoring period of the patient. The hemodynamic monitor saves the first dataset to a memory of the hemodynamic monitor. The method also includes receiving, at the hemodynamic monitor, a second dataset of the hemodynamic parameter of the patient from a second sensor throughout the monitoring period. The hemodynamic monitor saves the second dataset to the memory of the hemodynamic monitor. A processor assigns a first priority to the first dataset. The processor assigns a second priority to the second dataset. In a first step, the processor populates a data sequence with all of the values of the first dataset relative to time of the monitoring period. In a second step, the processor populates any segments of time missing a hemodynamic parameter value in the data sequence with a hemodynamic parameter value from a corresponding time segment in the second dataset after completion of the first step. The processor outputs the data sequence to a display.

In another aspect of the disclosure, a system for monitoring of a hemodynamic parameter of a patient includes a first hemodynamic sensor and a second hemodynamic sensor. The first hemodynamic sensor is configured to sense the hemodynamic parameter of the patient and produce a first dataset of the hemodynamic parameter of the patient. The second hemodynamic sensor is configured to sense the hemodynamic parameter of the patient and produce a second dataset of the hemodynamic parameter of the patient. A hemodynamic monitor is in communication with the first hemodynamic sensor and the second hemodynamic sensor. A system memory is configured to receive the first dataset and the second dataset from the hemodynamic monitor. The system memory also stores aggregating tool software code. The system also includes a hardware processor that is configured to execute the aggregating software code to assign a first priority to the first dataset, assign a second priority to the second dataset, and perform a first step by populating a data sequence with all of the values of the first dataset relative to time of a monitoring period. The hardware processor is also configured to execute the aggregating software code to perform a second step by populating any segments of time missing a hemodynamic parameter value in the data sequence with a hemodynamic parameter value from a corresponding time segment in the second dataset after completion of the first step. The hardware processor is also configured to execute the aggregating software code to output the data sequence after completion of the second step to a display in communication with the processor.

In another aspect of the disclosure, a method is disclosed for monitoring of a hemodynamic parameter of a patient. The method includes sensing the hemodynamic parameter of the patient with a first sensor and communicating a first dataset of the hemodynamic parameter to a hemodynamic monitor by the first sensor throughout a monitoring period of the patient. The hemodynamic monitor saves the first dataset to a memory of the hemodynamic monitor. The method also includes sensing the hemodynamic parameter of the patient with a second sensor and communicating a second dataset of the hemodynamic parameter to the hemodynamic monitor by the second sensor throughout the monitoring period. The hemodynamic monitor saves the second dataset to the memory of the hemodynamic monitor. A processor assigns a first priority to the first dataset and assigns a second priority to the second dataset. In a first step the processor populates a data sequence with all of the values of the first dataset relative to time of the monitoring period.

### BRIEF DESCRIPTION OF THE DRAWINGS

FIG. 1 is a block diagram illustrating an example hemodynamic monitoring system that includes a plurality of hemodynamic sensors connected to a patient.
FIG. 2 is a diagram illustrating hemodynamic data of a patient being transferred from a hemodynamic monitor to a computer for analysis.
FIG. 3 is a block diagram illustrating an example computer system for processing sensed hemodynamic data collected by the hemodynamic monitoring system of FIG. 1.
FIG. 4 is a diagram illustrating a user interface of the computer system and a table of hemodynamic data from the hemodynamic monitoring system.
FIG. 5 is a diagram of another embodiment of the user interface and the table of hemodynamic data from FIG. 4.
FIG. 6 is a table and graph illustrating two example outputs of hemodynamic data from the tables of FIGS 4 and 5.

### DETAILED DESCRIPTION

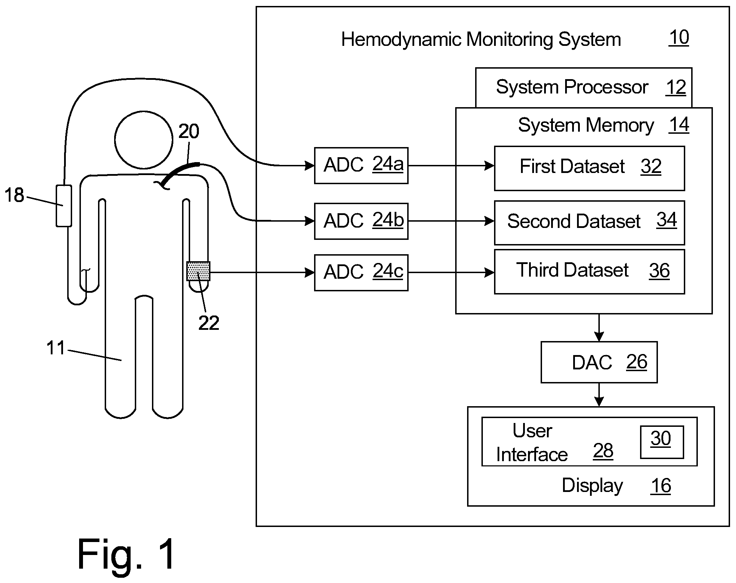

As described herein, a hemodynamic monitoring system includes a plurality of sensors configured to sense hemodynamic parameters of a patient. The plurality of sensors can include invasive sensors that are inserted through a heart of the patient to a pulmonary artery of the patient, non-invasive sensors that are attached over the skin of the patient (such as around a finger of the patient), and minimally invasive sensors that are inserted into a radial or femoral artery of the patient. The hemodynamic monitoring system simultaneously collects and stores hemodynamic data of the patient sensed by each of the plurality of sensors during a monitoring period. The hemodynamic data is transferred to another computer system that includes an aggregating tool. The aggregating tool includes a first step where the physician or a technician assigns a priority to each of the plurality of sensors. Based on the priority assigned to each of the plurality of sensors, the aggregating tool generates a data sequence for the monitoring period that is a hybrid of the hemodynamic data sensed by the plurality of sensors. The computer system is configured to output visual representation of the data sequence to a display. As the data sequence combines information from multiple different sensors into a single output, a physician can be more informed of a hemodynamic status, stability, or instability of the patient than by individually analyzing each output of the plurality of sensors. The hemodynamic monitoring system and aggregating tool is discussed in greater detail below with reference to FIGS. 1-6.

FIG. 1 is a block diagram of one example of hemodynamic monitoring system 10 that monitors patient 11 and simultaneously and concurrently collects multiple sets of hemodynamic data over a period of time from a plurality of sensors connected to patient 11. The multiple sets of hemodynamic data from the plurality of sensors will subsequently be processed to generate a single data sequence representative of a hemodynamic parameter of patient 11 for that period of time.

As illustrated in the example of FIG. 1, hemodynamic monitoring system 10 includes hemodynamic monitoring system 10, system processor 12, system memory 14, display 16, first sensor 18, second sensor 20, and third sensor 22. Hemodynamic monitoring system 10 can also include first analog-to-digital (ADC) converter 24a, second analog-to-digital (ADC) converter 24b, third analog-to-digital (ADC) converter 24c, and digital-to-analog (DAC) converter 26. Display 16 can include user interface 28 and control elements 30. System memory 14 compiles and stores first dataset 32 from first sensor 18, compiles and stores second dataset 32 from second sensor 20, and compiles and stores third dataset 36 from third sensor 22. Hemodynamic monitoring system 10 can be implemented within a patient care environment, such as an intensive care unit (ICU), an operating room (OR), an emergency room (ER), and ambulatory surgical center, or other patient care environment.

Hemodynamic monitoring system 10 can be an integrated hardware unit including system processor 12, system memory 14, display 16, and analog-to-digital (ADC) converters 24a-24c. In some examples, hemodynamic monitoring system 10 can also include digital-to-analog (DAC) converter 26. In other examples, any one or more components and/or described functionality of hemodynamic monitoring system 10 can be distributed among multiple hardware units. For instance, in some examples, display 16 can be a separate display device that is remote from and operatively coupled with hemodynamic monitoring system 10. In general, though illustrated and described in the example of FIG. 1 as an integrated hardware unit, it should be understood that hemodynamic monitoring system 10 can include any combination of devices and components that are electrically, communicatively, or otherwise operatively connected to perform functionality attributed herein to hemodynamic monitoring system 10. Display 16 provides user interface 28, which includes control elements 30 that enable user interaction with hemodynamic monitoring system 10 and/or other components of hemodynamic monitoring system 10.

System processor 12 is a hardware processor configured to execute software code to compile and save each of first dataset 32, second dataset 34, and third dataset 36 to system memory 14. System processor 12 can also communicate first dataset 32, second dataset 34, and third dataset 36 to display 16 so a physician can visually review first dataset 32, second dataset 34, and third dataset 36 in real time. Examples of system processor 12 can include any one or more of a microprocessor, a controller, a digital signal processor (DSP), an application specific integrated circuit (ASIC), a field-programmable gate array (FPGA), or other equivalent discrete or integrated logic circuitry.

System memory 14 can be configured to store information within hemodynamic monitoring system 10 during operation. System memory 14, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 14 can include volatile and non-volatile computer-readable memories. Examples of volatile memories can include random access memories (RAM), dynamic random access memories (DRAM), static random access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 16 can be a liquid crystal display (LCD), a light-emitting diode (LED) display, an organic light-emitting diode (OLED) display, or other display device suitable for providing information to users in graphical form. User interface 28 can include graphical and/or physical control elements 30 that enable user input to interact with hemodynamic monitoring system 10 and/or other components of hemodynamic monitoring system 10. In some examples, user interface 28 can take the form of a graphical user interface (GUI) that presents graphical control elements 30 presented at, e.g., a touch-sensitive and/or presence sensitive display screen of display 16. In such examples, user input can be received in the form of gesture input, such as touch gestures, scroll gestures, zoom gestures, or other gesture input. In certain examples, user interface 28 can take the form of and/or include physical control elements, such as a physical buttons, keys, knobs, or other physical control elements configured to receive user input to interact with components of hemodynamic monitoring system 10. User interface 28 can include a speaker that allows hemodynamic monitoring system 10 the ability to generate an audible alarm.

First sensor 18 can be attached to patient 11 to sense first hemodynamic data of patient 11. First sensor 18 is operatively connected to hemodynamic monitoring system 10 (e.g., electrically and/or communicatively connected via wired or wireless connection, or both) to provide the sensed first hemodynamic data to system memory 14 of hemodynamic monitoring system 10. In some examples, first sensor 18 provides the first hemodynamic data of patient 11 to hemodynamic monitoring system 10 as an analog signal, which is converted by first ADC 24a to first digital hemodynamic data. The first digital hemodynamic data is compiled by system processor 12 and saved and stored to system memory 14 as first dataset 32. In other examples, first sensor 18 can provide the sensed first hemodynamic data to hemodynamic monitoring system 10 in digital form, in which case hemodynamic monitoring system 10 may not include or utilize first ADC 24a.

In the example of FIG. 1, first sensor 18 is a minimally invasive hemodynamic sensor configured to sense hemodynamic parameters of patient 11 in a minimally invasive manner. For instance, first sensor 18 can be attached to patient 11 via a radial arterial catheter inserted into an arm of patient 11. In other examples, first sensor 18 can be attached to patient 11 via a femoral arterial catheter inserted into a leg of patient 11. Such minimally invasive techniques can enable first hemodynamic sensor 11 to provide substantially continuous beat-to-beat monitoring of the hemodynamic parameters of patient 11 over an extended period of time, such as minutes, hours, or days. The hemodynamic parameters of patient 11 can include but are not limited to arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility.

Second sensor 20 can be attached to patient 11 to sense second hemodynamic data of patient 11. Second sensor 20 is operatively connected to hemodynamic monitoring system 10 (e.g., electrically and/or communicatively connected via wired or wireless connection, or both) to provide the sensed second hemodynamic data to system memory 14 of hemodynamic monitoring system 10. In some examples, second sensor 20 provides the second hemodynamic data of patient 11 to hemodynamic monitoring system 10 as an analog signal, which is converted by second ADC 24b to second digital hemodynamic data. The second digital hemodynamic data is compiled by system processor 12 and saved and stored to system memory 14 as second dataset 34. In other examples, second sensor 20 can provide the sensed second hemodynamic data to hemodynamic monitoring system 10 in digital form, in which case hemodynamic monitoring system 10 may not include or utilize second ADC 24b.

In the example of FIG. 1, second sensor 20 is an invasive hemodynamic sensor 20 configured to sense the hemodynamic parameters of patient 11 in an invasive manner. For instance, second sensor 20 can be attached to patient 11 via a pulmonary artery catheter inserted through the heart of patient 11. Such invasive techniques can enable second sensor 20 to provide substantially continuous monitoring of the hemodynamic parameters of patient 11 over an extended period of time, such as minutes, hours, or days. Second sensor 20 and first sensor 18 can each sense and communicate data regarding the hemodynamic parameters of patient 11 to hemodynamic monitoring system 10 concurrently to each other throughout the period of time that hemodynamic monitoring system 10 is monitoring patient 11.

Third sensor 22 can be attached to patient 11 to sense third hemodynamic data of patient 11. Third sensor 22 is operatively connected to hemodynamic monitoring system 10 (e.g., electrically and/or communicatively connected via wired or wireless connection, or both) to provide the sensed third hemodynamic data to system memory 14 of hemodynamic monitoring system 10. In some examples, third sensor 22 provides the third hemodynamic data of patient 11 to hemodynamic monitoring system 10 as an analog signal, which is converted by third ADC 24c to third digital hemodynamic data. The third digital hemodynamic data is compiled by system processor 12 and saved and stored to system memory 14 as third dataset 36. In other examples, third sensor 22 can provide the sensed third hemodynamic data to hemodynamic monitoring system 10 in digital form, in which case hemodynamic monitoring system 10 may not include or utilize third ADC 24c.

In the example of FIG. 1, third hemodynamic sensor 22 is a non-invasive hemodynamic sensor 22 configured to sense the hemodynamic parameters of patient 11 in a non-invasive manner. For instance, third sensor 22 can be attached non-invasively to an extremity of patient 11, such as a wrist, an arm, a finger, an ankle, a toe, or other extremity of patient 11. As such, third sensor 22 can take the form of a small and lightweight hemodynamic sensor suitable for extended wear by patient 11 to provide substantially continuous beat-to-beat monitoring of the hemodynamic parameters of patient 11 over an extended period of time, such as minutes, hours, or days. Third sensor 22, second sensor 20, and first sensor 18 can each sense and communicate data regarding the hemodynamic parameters of patient 11 to hemodynamic monitoring system 10 concurrently to each other throughout the period of time that hemodynamic monitoring system 10 is monitoring patient 11.

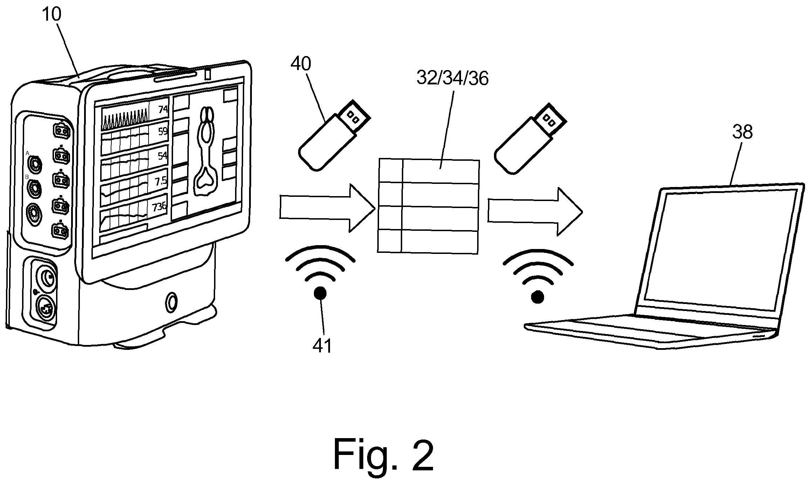

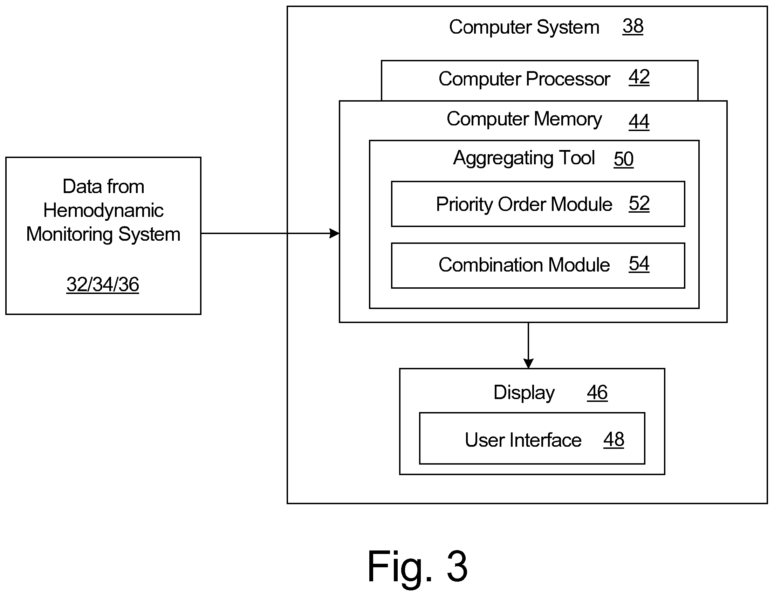

Through first sensor 18, second sensor 20, and third sensor 22, hemodynamic monitoring system 10 collects three distinct datasets 32/34/36 measuring the same hemodynamic parameter. While first sensor 18, second sensor 20, and third sensor 22 are all measuring the same hemodynamic parameter of patient 11, differences can exist between the first hemodynamic data saved to first dataset 32, the second hemodynamic data saved to second dataset 34, and the third hemodynamic data saved to third dataset 36. These differences in data can be attributed to the different sampling rates, accuracy levels, noise levels, time lag, and methods that invasive, minimally invasive, and non-invasive sensors detect the hemodynamic parameter. For example, invasive hemodynamic sensors, such as second sensor 20, is considered more reliable by some practitioners over non-invasive or minimally invasive hemodynamic sensors. However, invasive hemodynamic sensors can experience more lag or a slower sampling rate than non-invasive or minimally invasive hemodynamic sensors. Due to the differences between these hemodynamic sensors, some physicians will weigh the differences between the different kinds of hemodynamic sensors and limit monitoring of the hemodynamic parameter to a particular kind of hemodynamic sensor while excluding the other kinds of hemodynamic monitoring systems. As discussed above, hemodynamic monitoring system 10 can utilize a plurality of hemodynamic sensors of various kinds concurrently. Normally a practitioner would not utilize such a large number of sensors to measure the same hemodynamic parameter of patient 11 because the large amount of concurrent data gathered by the plurality of sensors is mentally overwhelming. However, as discussed below with reference to FIGS. 2 and 3, the present disclosure includes an aggregating tool that can generate an aggregated data sequence of the sensed parameter of patient 11 that is based on first dataset 32, second dataset 34, and third dataset 36. This aggregated data sequence provides a more complete measurement of the sensed parameter of patient 11 than is provided alone by first dataset 32, second dataset 34, or third dataset 36, but is more readily comprehensible mentally by a physician or practitioner than reading all of first dataset 32, second dataset 34, or third dataset 36 together.

FIG. 2 is a diagram illustrating first dataset 32, second dataset 34, and third dataset 36 being transferred from hemodynamic monitoring system 10 to computer system 38. As shown in FIG. 2, first dataset 32, second dataset 34, and third dataset 36 can be transferred to computer system 38 physically or wirelessly. In the case of physical transference, first dataset 32, second dataset 34, and third dataset 36 can be transferred from hemodynamic monitoring system 10 to a portable memory device, such as flash drive 40, and then transferred from the portable memory device to computer system 38. In the case of wireless transmission, first dataset 32, second dataset 34, and third dataset 36 can be transferred from hemodynamic monitoring system 10 to computer system 38 through a wireless network, such as through a wireless fidelity network (e.g., Wi-Fi) 41, through a broadband wireless network, or through a Bluetooth connection between hemodynamic monitoring system 10 and computer system 38. While not illustrated, hemodynamic monitoring system 10 can also be wired, such as through an ethernet connection, directly to computer system 38, or wired to a network connected to computer system 38.

As discussed below with reference to FIG. 3, the aggregating tool is executed on computer system 38 to produce the aggregated data sequence of the sensed parameter of patient 11 based on first dataset 32, second dataset 34, and third dataset 36. While the example of FIGS. 2 and 3 disclose the aggregating tool being executed on computer system 38, in other examples, the aggregating tool can be saved to system memory 14 of hemodynamic monitoring system 10 and executed by system processor 12 of hemodynamic monitoring system 10 to generate the aggregated data sequence, which can then be displayed in real time to display 16 and/or saved to system memory 14 for future analysis.

FIG. 3 is a block diagram illustrating an example of computer system 38. In the example of FIG. 3, computer system 38 includes computer processor 42, computer memory 44, and display 46 with user interface 48. Computer memory 44 includes aggregating tool 50 with priority order module 52 and combination module 54.

Computer system 38 can be an integrated hardware unit including computer processor 42, computer memory 44, and display 46. In other examples, any one or more components and/or described functionality of computer system 38 can be distributed among multiple hardware units. For instance, in some examples, display 46 can be a separate display device that is remote from and operatively coupled with computer system 38. In general, though illustrated and described in the example of FIG. 3 as an integrated hardware unit, it should be understood that computer system 38 can include any combination of devices and components that are electrically, communicatively, or otherwise operatively connected to perform functionality attributed herein to computer system 38. Display 46 provides user interface 48 which includes control elements that enable user interaction with computer system 38. A user can interact with user interface 48 in display 46 through a mouse, a keyboard, and/or through touch features of display 46.

Computer processor 42 is a hardware processor configured to execute software code saved to computer memory 44, including aggregating tool 50, to generate the aggregated data sequence from first dataset 32, second dataset 34, and third dataset 36. Computer processor 42 can also communicate the aggregated data sequence to display 46 and store the aggregated data sequence to computer memory 44. Examples of computer processor 42 can include any one or more of a microprocessor, a controller, a digital signal processor (DSP), an application specific integrated circuit (ASIC), a field-programmable gate array (FPGA), or other equivalent discrete or integrated logic circuitry.

Computer memory 44 can be configured to store information computer system 38 during operation. Computer memory 44, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). Computer memory 44 can include volatile and non-volatile computer-readable memories. Examples of volatile memories can include random access memories (RAM), dynamic random access memories (DRAM), static random access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 46 can be a liquid crystal display (LCD), a light-emitting diode (LED) display, an organic light-emitting diode (OLED) display, or other display device suitable for providing information to users in graphical form. User interface 48 can include graphical and/or physical control elements that enable user input to interact with computer system 38 and/or other components of computer system 38. In some examples, user interface 48 can take the form of a graphical user interface (GUI) that presents graphical control elements presented at, e.g., a touch-sensitive and/or presence sensitive display screen of display 46. In such examples, user input can be received in the form of gesture input, such as touch gestures, scroll gestures, zoom gestures, or other gesture input. In certain examples, user interface 48 can take the form of and/or include physical control elements, such as a physical buttons, keys, knobs, or other physical control elements configured to receive user input to interact with components of computer system 38.

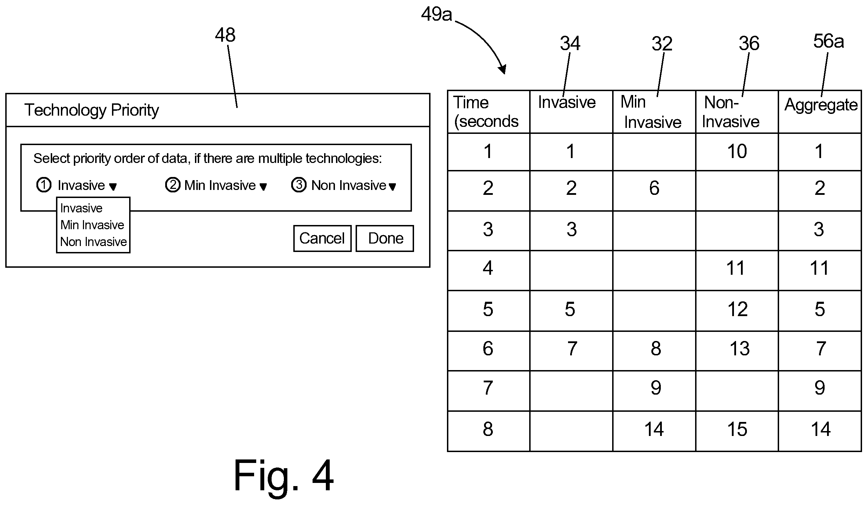

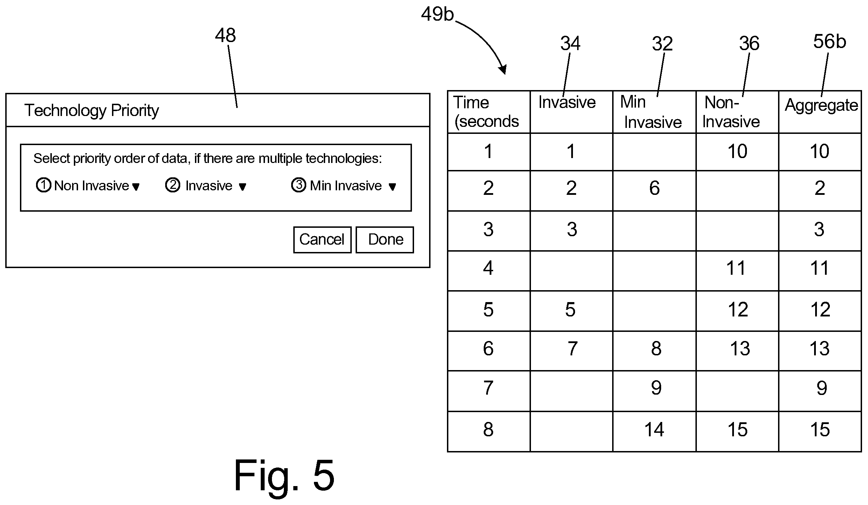

During operation of computer system 38, computer processor 42 receives first dataset 32, second dataset 34, and third dataset 36 from hemodynamic monitoring system 10 and saves first dataset 32, second dataset 34, and third dataset 36 to computer memory 44. Computer processor 42 then executes priority order module 52 of aggregating tool 50. Priority order module is a software module or algorithm that is executed by computer processor 42 and allows the user to assign an order of priority to first dataset 32, second dataset 34, and third dataset 36. As discussed below in greater detail with reference to FIGS. 4 and 5, priority order module 52 can generate a prompt in user interface 48 instructing a user to assign a priority order to first dataset 32, second dataset 34, and third dataset 36. In some examples, the user can save a default priority order to computer memory 44, such that priority order module 52 can skip the prompt to user interface 48 and proceed by automatically loading the default priority.

Priority order module 52 then applies the priority order to first dataset 32, second dataset 34, and third dataset 36. Priority order module 52 can apply the priority order to first dataset 32, second dataset 34, and third dataset 36 by applying a specific address indicative of a place within the order, assigning a label indicative of a variable or place within a function, or any other software means or method known for assigning an order of priority between multiple datasets. After executing priority order module 52, computer processor 42 can execute combination module 54 of aggregating tool 50. Combination module 54 is a software module or algorithm that is executed by computer processor 42 to combine measured values from first dataset 32, second dataset 34, and third dataset 36 into the aggregated data sequence of the sensed parameter of patient 11. Computer processor 42 can then output the aggregated data sequence to display 46 of computer system 38 and save the aggregated data sequence to computer memory 44. How combination module 54 combines the measured values from first dataset 32, second dataset 34, and third dataset 36 into the aggregated data sequence is discussed below with reference to FIGS. 4 and 5.

FIG. 4 is a diagram illustrating user interface 48 of computer system 38 and first table 49a. In the example of FIG. 4, first table 49a includes a first column on the left-hand side indicating time, a second column with the values of second dataset 34 measured by second (invasive) sensor 20, a third column with the values of first dataset 32 measured by first (minimally invasive) sensor 18, a fourth column with the values of third dataset 36 measured by third (non-invasive) sensor 22, and a fifth column on the right-hand side containing the values of first aggregated data sequence 56a.

As shown in the example of user interface 48 in FIG. 4, when computer processor 42 executes aggregating tool 50, user interface 48 instructs the user to assign a priority value to first sensor 18, second sensor 20, and third sensor 22. In the example of FIGS. 1 and 4, the user has assigned first priority to second sensor 20 (which is an invasive sensor), the user has assigned second priority to first sensor 18 (which is a minimally invasive sensor), and the user has assigned third priority to third sensor 22 (which is a non-invasive sensor).

As shown in first table 49a, second sensor 20 has provided to second dataset 34 some values over time, in particular, to seconds 1-3 and 5-6 of the time period observed by hemodynamic monitoring system 10. However, there are some gaps in second dataset 34 where second sensor 20 was unable to provide a measurement for the sensed parameter. The sensed parameter in this example can be one of the hemodynamic parameters of patient 11 discussed above with reference to FIG. 1. In particular second dataset 34 is missing values for seconds 4, 7, and 8.

First sensor 18 has provided to first dataset 32 some values over time, in particular, to seconds 2 and 6-8 of the time period observed by hemodynamic monitoring system 10. However, there are some gaps in first dataset 32 where first sensor 18 was unable to provide a measurement for the sensed parameter. In particular, first dataset 32 is missing values for seconds 1 and 3-5.

Third sensor 22 has provided to third dataset 36 some values over time, in particular, to seconds 1, 4-6, and 8 of the time period observed by hemodynamic monitoring system 10. However, there are some gaps in third dataset 36 where third sensor 22 was unable to provide a measurement for the sensed parameter. In particular, third dataset 36 is missing values for seconds 2-3 and 7.

Aggregating tool 50 populates the aggregated data sequence by first including values from the dataset given first priority by the user. After the values from the dataset given first priority have been populated into the aggregated data sequence, if a time segment in the aggregated data sequence is missing a value, aggregating tool 50 will populate values for those empty time segments from the dataset given second priority by the user. If any time segments remain empty in the aggregated data sequence after populating the aggregated data sequence with values from the dataset given second priority, aggregating tool 50 will populate values for those empty time segments from the dataset given third priority by the user.

In the example of FIGS. 1 and 4, the user has assigned first priority to second sensor 20 and second dataset 34. Aggregating tool 50 first populates first aggregated data sequence 56a with all of the values from second dataset 34. Since second dataset 34 did not have any measured values for seconds 4, 7, and 8 of the time period hemodynamic monitoring system 10 monitored patient 11, aggregating tool 50 goes to first dataset 32 to fill seconds 4, 7, and 8 since the user has assigned first dataset 32 second priority. First dataset 32 has values for seconds 7 and 8, which are added as values for seconds 7 and 8 of first aggregated data sequence 56a. Since first dataset 32 and second dataset 34 both lack a value for second 4 of the time period hemodynamic monitoring system 10 monitored patient 11, aggregating tool 50 goes to third dataset 36 to fill second 4. Third dataset 36 does include a value for second 4, and that value is added by aggregating tool 50 to first aggregated data sequence 56a.

Once aggregating tool 50 has added values from second dataset 34, first dataset 32, and third dataset 36 to first aggregated data sequence 56a in order of the priority assigned by the user, first aggregated data sequence 56a is completed and outputted to display 46. As evident in first table 49a of FIG. 4, first aggregated data sequence 56a provides a more complete sequence of measured the hemodynamic parameter of patient 11 with respect to time than provided by first dataset 32 alone, second dataset 34 alone, or third dataset 36 alone. First aggregated data sequence 56a is also simpler to follow than trying to analyze first dataset 32, second dataset 34, and third dataset 36 together simultaneously.

While the time period shown in the example of FIG. 4 is only 8 seconds in length (deliberately short to ease illustration of aggregating tool 50), the time period can be several minutes, hours, or days in length, with thousands, tens of thousands, or hundreds of thousands of values from each of first sensor 18, second sensor 20, and third sensor 22. While aggregating tool 50 can be applied retroactively to compiled first dataset 32, second dataset 34, and third dataset 36 after the time period of observing patient 11 has been completed by hemodynamic monitoring system 10, in some examples, aggregating tool 50 can be applied in real time to load values from first sensor 18, second sensor 20, and third sensor 22 into first aggregated data sequence 56a as those values are measured and received by hemodynamic monitoring system 10.

In the example of FIG. 4, since there are three sensors (first sensor 18, second sensor 20, and third sensor 22), and since there are three positions of priority that the user can assign, there are six possible aggregated data sequences that aggregating tool 50 can produce from first dataset 32, second dataset 34, and third dataset 36. FIG. 4 shows one of the six possible aggregated data sequences and FIG. 5 shows a second possible aggregated data sequence.

FIG. 5 is a diagram illustrating user interface 48 of computer system 38 and second table 49b. In the example of FIG. 5, second table 49b includes a first column on the left-hand side indicating time, a second column with the values of second dataset 34 measured by second (invasive) sensor 20, a third column with the values of first dataset 32 measured by first (minimally invasive) sensor 18, a fourth column with the values of third dataset 36 measured by third (non-invasive) sensor 22, and a fifth column on the right-hand side containing the valves of second aggregated data sequence 56b. In the example of FIG. 5, first dataset 32, second dataset 34, and third dataset 36 are the same in second table 49b as first table 49a shown in FIG. 4. The only difference between second table 49b and first table 49a is that second table 49b includes second aggregated data sequence 56b whereas first table 49b includes first aggregated data sequence 56a.

As shown in the example of user interface 48 in FIG. 5, when computer processor 42 executes aggregating tool 50, user interface 48 instructs the user to assign a priority value to first sensor 18, second sensor 20, and third sensor 22. In the example of FIGS. 1 and 5, the user has assigned first priority to third sensor 22 (which is the non-invasive sensor), the user has assigned second priority to second sensor 20 (which is the invasive sensor), and the user has assigned third priority to first sensor 18 (which is the minimally invasive sensor).

As shown in second table 49b of FIG. 5 and previously discussed above with reference to FIG. 4, third sensor 22 has provided to third dataset 36 some values over time, in particular, to seconds 1, 4-6, and 8 of the time period observed by hemodynamic monitoring system 10. However, there are some gaps in third dataset 36 where third sensor 22 was unable to provide a measurement for the sensed parameter. In particular, third dataset 36 is missing values for seconds 2-3 and 7.

Second sensor 20 has provided to second dataset 34 some values over time, in particular, to seconds 1-3 and 5-6 of the time period observed by hemodynamic monitoring system 10. However, there are some gaps in second dataset 34 where second sensor 20 was unable to provide a measurement for the sensed parameter. The sensed parameter in this example can be one of the hemodynamic parameters of patient 11 discussed above with reference to FIG. 1. In particular, second dataset 34 is missing values for seconds 4, 7, and 8.

First sensor 18 has provided to first dataset 32 some values over time, in particular, to seconds 2 and 6-8 of the time period observed by hemodynamic monitoring system 10. However, there are some gaps in first dataset 32 where first sensor 18 was unable to provide a measurement for the sensed parameter. In particular, first dataset 32 is missing values for seconds 1 and 3-5.

In the example of FIGS. 1 and 5, the user has assigned first priority to third sensor 22 and third dataset 36. Aggregating tool 50 first populates second aggregated data sequence 56b with all of the values from third dataset 36. Since third dataset 36 did not have any measured values for seconds 2, 3, and 7 of the time period hemodynamic monitoring system 10 monitored patient 11, aggregating tool 50 goes to second dataset 34 to fill seconds 2, 3, and 7 since the user has assigned second dataset 34 second priority. Second dataset 34 has values for seconds 2 and 3, which are added as values for seconds 2 and 3 of second aggregated data sequence 56b. Since second dataset 34 and third dataset 36 both lack a value for second 7 of the time period hemodynamic monitoring system 10 monitored patient 11, aggregating tool 50 goes to first dataset 32 to fill second 7. First dataset 32 does include a value for second 7, and that value is added by aggregating tool 50 to second aggregated data sequence 56b.

Once aggregating tool 50 has added values from third dataset 36, second dataset 34, and first dataset 32 to second aggregated data sequence 56b in order of the priority assigned by the user, second aggregated data sequence 56b is completed and outputted to display 46. As evident in second table 49b of FIG. 5, second aggregated data sequence 56b provides a more complete sequence of the measured hemodynamic parameter of patient 11 with respect to time than provided by first dataset 32 alone, second dataset 34 alone, or third dataset 36 alone. Second aggregated data sequence 56b is also simpler to follow than trying to analyze first dataset 32, second dataset 34, and third dataset 36 together simultaneously.

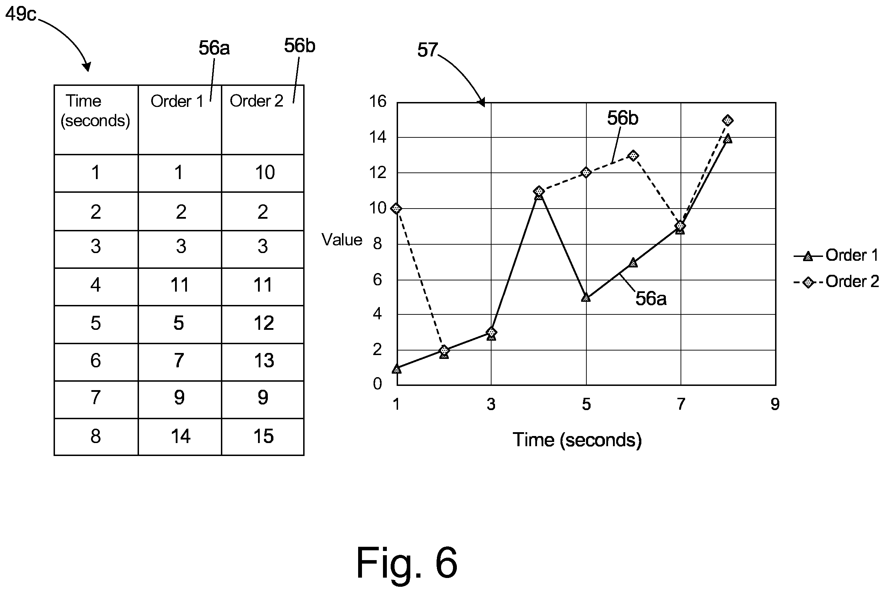

FIG. 6 discloses third table 49c and graph 57. Third table 49c shows the values for first aggregated data sequence 56a from the example of FIG. 4 and the values of second aggregated data sequence 56b from the example of FIG. 5. Graph 57 shows plots for both first aggregated data sequence 56a and second aggregated data sequence 56b.

While first aggregated data sequence 56a and second aggregated data sequence 56b are based on exactly the same three datasets from hemodynamic monitoring system 10, first aggregated data sequence 56a places more emphasis on data from one kind of sensor (second sensor 20) and second aggregated data sequence 56b places emphasis on data from another kind of sensor (third sensor 22). Even though first aggregated data sequence 56a emphasizes data from second sensor 20, first aggregated data sequence 56a takes advantage of the data from first sensor 18 and the data from third sensor 22 when second sensor 20 falls short. Similarly, even though second aggregated data sequence 56b emphasizes data from third sensor 22, second aggregated data sequence 56b takes advantage of the data from first sensor 18 and the data from second sensor 20 when third sensor 22 falls short.

The present disclosure provides a method for monitoring of a hemodynamic parameter of a patient 11. The method includes sensing the hemodynamic parameter of the patient with a first sensor 18. The first sensor 18 communicates a first dataset 32 of the hemodynamic parameter to a hemodynamic monitor throughout a monitoring period of the patient 11. The hemodynamic monitor saves the first dataset 32 to a memory 14 of the hemodynamic monitor. The method also includes sensing the hemodynamic parameter of the patient with a second sensor 20. The second sensor 20 communicates a second dataset 34 of the hemodynamic parameter to the hemodynamic monitor throughout the monitoring period. The hemodynamic monitor saves the second dataset 34 to the memory of the hemodynamic monitor. A processor assigns a first priority to the first dataset 32. The processor assigns a second priority to the second dataset 34. In a first step, the processor 12 populates a data sequence with all of the values of the first dataset 32 relative to time of the monitoring period. In a second step, the processor 12 populates any segments of time missing a hemodynamic parameter value in the data sequence with a hemodynamic parameter value from a corresponding time segment in the second dataset 34 after completion of the first step. The processor outputs the data sequence to a display 16.

The method of the preceding paragraph can optionally include, additionally and/or alternatively, any one or more of the following features, configurations and/or additional components in the paragraphs below.

In an embodiment of the foregoing method, the first sensor is an invasive hemodynamic sensor 20, a minimally invasive hemodynamic sensor 18, or a non-invasive hemodynamic sensor 22; and wherein the second sensor is an invasive hemodynamic sensor 20, a minimally invasive hemodynamic sensor 18, or a non-invasive hemodynamic sensor.

In an embodiment of the foregoing method, the method further comprises: sensing the hemodynamic parameter of the patient with a third sensor 22 and communicating a third dataset 36 of the hemodynamic parameter to the hemodynamic monitor by the third sensor 36 throughout the monitoring period of the patient 11; saving, by the hemodynamic monitor, the third dataset 36 to the memory 14 of the hemodynamic monitor; assigning, by a processor 12, a third priority to the third dataset 36; populating through a third step, by the processor 12, any segments of time missing a hemodynamic parameter value in the data sequence with a hemodynamic parameter value from a corresponding time segment in the third dataset 36 after completion of the second step; and outputting, by the processor, the data sequence to a display 16.

In an embodiment of the foregoing method, the third sensor is an invasive hemodynamic sensor 20, a minimally invasive hemodynamic sensor 18, or a non-invasive hemodynamic sensor 22.

In an embodiment of the foregoing method, the processor 12 is a computer processor 42 of a device 38 physically separate from the hemodynamic monitor.

In an embodiment of the foregoing method, the processor 12 is a processor of the hemodynamic monitor.

In an embodiment of the foregoing method, the assigning by the processor 12 the first priority to the first dataset is in response to a first input from a user through a user interface 28 in communication with the processor 12; and wherein the assigning by the processor the second priority to the second dataset is in response to a second input from the user through the user interface 28.

In an embodiment of the foregoing method, the hemodynamic parameter is at least one of arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility of the patient 11.

The present disclosure also provides a method for monitoring of a hemodynamic parameter of a patient 11, the method comprising: receiving, at a hemodynamic monitor, a first dataset 32 of a hemodynamic parameter of the patient 11 from a first sensor 18 throughout a monitoring period of the patient 11; saving, by the hemodynamic monitor, the first dataset 32 to a memory 14 of the hemodynamic monitor; receiving, at a hemodynamic monitor, a second dataset 34 of the hemodynamic parameter of the patient from a second sensor 20 throughout the monitoring period; saving, by the hemodynamic monitor, the second dataset 34 to the memory 14 of the hemodynamic monitor; assigning, by a processor 12, a first priority to the first dataset 32; assigning, by the processor 12, a second priority to the second dataset 34; populating through a first step, by the processor 12, a data sequence with all of the values of the first dataset 32 relative to time of the monitoring period; populating through a second step, by the processor 12, any segments of time missing a hemodynamic parameter value in the data sequence with a hemodynamic parameter value from a corresponding time segment in the second dataset 34 after completion of the first step; and outputting, by the processor 12, the data sequence to a display 16.

The method of the preceding paragraph can optionally include, additionally and/or alternatively, any one or more of the following features, configurations and/or additional components in the paragraphs below.

In an embodiment of the foregoing method, the method includes the step of communicating the first dataset 32 of the hemodynamic parameter to the hemodynamic monitor by the first sensor 18 throughout a monitoring period of the patient 11 and/or the step of communicating the second dataset 34 of the hemodynamic parameter to the hemodynamic monitor by the second sensor 20 throughout a monitoring period of the patient 11.

In an embodiment of the foregoing method, the first sensor is an invasive hemodynamic sensor 20, a minimally invasive hemodynamic sensor 18, or a non-invasive hemodynamic sensor 22; and wherein the second sensor is an invasive hemodynamic sensor 20, a minimally invasive hemodynamic sensor 18, or a non-invasive hemodynamic sensor.

In an embodiment of the foregoing method, the method further comprises: receiving, at the hemodynamic monitor, a third dataset 36 of the hemodynamic parameter of the patient from a third sensor 22 throughout the monitoring period of the patient; saving, by the hemodynamic monitor, the third dataset 36 to the memory 14 of the hemodynamic monitor; assigning, by a processor 12, a third priority to the third dataset 36; populating through a third step, by the processor 12, any segments of time missing a hemodynamic parameter value in the data sequence with a hemodynamic parameter value from a corresponding time segment in the third dataset 36 after completion of the second step; and outputting, by the processor, the data sequence to a display 16.

In an embodiment of the foregoing method, the method includes the step of communicating the third dataset 36 of the hemodynamic parameter to the hemodynamic monitor by the third sensor 22 throughout a monitoring period of the patient 11.

In an embodiment of the foregoing method, the third sensor is an invasive hemodynamic sensor 20, a minimally invasive hemodynamic sensor 18, or a non-invasive hemodynamic sensor 22.

In an embodiment of the foregoing method, the processor 12 is a computer processor 42 of a device 38 physically separate from the hemodynamic monitor.

In an embodiment of the foregoing method, the processor 12 is a processor of the hemodynamic monitor.

In an embodiment of the foregoing method, the assigning by the processor 12 the first priority to the first dataset is in response to a first input from a user through a user interface 28 in communication with the processor 12; and wherein the assigning by the processor the second priority to the second dataset is in response to a second input from the user through the user interface 28.

In an embodiment of the foregoing method, the hemodynamic parameter is at least one of arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility of the patient 11.

The present disclosure also provides a method for monitoring of a hemodynamic parameter of a patient 11, the method comprising: assigning, by a processor 12, a first priority to a first dataset 32, wherein the first dataset 32 is a first dataset 32 of a hemodynamic parameter of a patient 11 sensed with a first sensor 18, communicated to the hemodynamic monitor by the first sensor 18 throughout a monitoring period of the patient 11 and saved, by the hemodynamic monitor, to a memory of the hemodynamic monitor; assigning, by the processor 12, a second priority to a second dataset 34, wherein the second dataset 34 is a second dataset 34 of a hemodynamic parameter of a patient 11 sensed with a second sensor 20, communicated to the hemodynamic monitor by the second sensor 20 throughout a monitoring period of the patient 11 and saved, by the hemodynamic monitor, to a memory of the hemodynamic monitor; populating through a first step, by the processor 12, a data sequence with all of the values of the first dataset 32 relative to time of the monitoring period; populating through a second step, by the processor 12, any segments of time missing a hemodynamic parameter value in the data sequence with a hemodynamic parameter value from a corresponding time segment in the second dataset 34 after completion of the first step; and outputting, by the processor 12, the data sequence to a display 16. This method can optionally include, *mutatis mutandis*, any of the features of the methods described in the preceding paragraphs.

In an embodiment of the foregoing method, the method further comprises: assigning, by a processor 12, a third priority to a third dataset 36, wherein the third dataset 36 is a third dataset 36 of a hemodynamic parameter of a patient 11 sensed with a third sensor 22, communicated to the hemodynamic monitor by the third sensor 22 throughout a monitoring period of the patient 11 and saved, by the hemodynamic monitor, to a memory of the hemodynamic monitor; populating through a third step, by the processor 12, any segments of time missing a hemodynamic parameter value in the data sequence with a hemodynamic parameter value from a corresponding time segment in the third dataset 36 after completion of the second step; and outputting, by the processor, the data sequence to a display 16.

The present disclosure provides a system 10 for monitoring of a hemodynamic parameter of a patient 11 which includes a first hemodynamic sensor 18 and a second hemodynamic sensor 20. The first hemodynamic sensor 18 is configured to sense the hemodynamic parameter of the patient and produce a first dataset 32 of the hemodynamic parameter of the patient. The second hemodynamic sensor 20 is configured to sense the hemodynamic parameter of the patient and produce a second dataset 34 of the hemodynamic parameter of the patient. A hemodynamic monitor is in communication with the first hemodynamic sensor and the second hemodynamic sensor. A system memory 14 is configured to receive the first dataset 32 and the second dataset 34 from the hemodynamic monitor. The system memory also stores aggregating tool software code. The system also includes a hardware processor 12 that is configured to execute the aggregating software code to assign a first priority to the first dataset 32, assign a second priority to the second dataset 34, and perform a first step by populating a data sequence with all of the values of the first dataset relative to time of a monitoring period. The hardware processor 12 is also configured to execute the aggregating software code to perform a second step by populating any segments of time missing a hemodynamic parameter value in the data sequence with a hemodynamic parameter value from a corresponding time segment in the second dataset after completion of the first step. The hardware processor is also configured to execute the aggregating software code to output the data sequence after completion of the second step to a display 16 in communication with the processor 12.

The system of the preceding paragraph can optionally include, additionally and/or alternatively, any one or more of the following features, configurations and/or additional components in the paragraphs below.

In an embodiment of the foregoing system, the first hemodynamic sensor is a non-invasive hemodynamic sensor 22 that is attachable to an extremity of the patient, wherein the first hemodynamic sensor is a minimally invasive hemodynamic sensor 18 connected to a catheter that is inserted into an extremity of the patient, or wherein the first hemodynamic pressure sensor is an invasive hemodynamic pressure sensor 20 connected to a cardiac catheter that is inserted into a heart of the patient.

In an embodiment of the foregoing system, the second hemodynamic sensor is a non-invasive hemodynamic sensor 22 that is attachable to an extremity of the patient, wherein the second hemodynamic sensor is a minimally invasive hemodynamic sensor 18 connected to a catheter that is inserted into an extremity of the patient, or wherein the second hemodynamic pressure sensor is an invasive hemodynamic pressure sensor 20 connected to a cardiac catheter that is inserted into a heart of the patient.

In an embodiment of the foregoing system, the system further comprises: a third hemodynamic sensor in communication with the hemodynamic monitor and configured to sense the hemodynamic parameter of the patient and produce a third dataset 36 of the hemodynamic parameter of the patient, wherein the system memory 14 configured to receive the third dataset from the hemodynamic monitor, and wherein the hardware processor 12 is configured to execute the aggregating software code to: assign a third priority to the third dataset 36; perform a third step by populating any segments of time missing a hemodynamic parameter value in the data sequence with a hemodynamic parameter value from a corresponding time segment in the third dataset after completion of the second step; and outputting the data sequence after completion of the third step to the display 16.

In an embodiment of the foregoing system, the third hemodynamic sensor is a non-invasive hemodynamic sensor 22 that is attachable to an extremity of the patient, wherein the third hemodynamic sensor is a minimally invasive hemodynamic sensor 18 connected to a catheter that is inserted into an extremity of the patient, or wherein the third hemodynamic pressure sensor is an invasive hemodynamic pressure sensor 20 connected to a cardiac catheter that is inserted into a heart of the patient.

In an embodiment of the foregoing system, the system further comprises a computer system 38 separate from the hemodynamic monitor and comprising the system memory 44 and the hardware processor 42.

In another aspect of the disclosure, a method is provided for monitoring of a hemodynamic parameter of a patient 11. The method includes sensing the hemodynamic parameter of the patient 11 with a first sensor 18 and communicating a first dataset 32 of the hemodynamic parameter to a hemodynamic monitor by the first sensor 18 throughout a monitoring period of the patient 11. The hemodynamic monitor saves the first dataset 32 to a memory 14 of the hemodynamic monitor. The method also includes sensing the hemodynamic parameter of the patient with a second sensor 20 and communicating a second dataset 34 of the hemodynamic parameter to the hemodynamic monitor by the second sensor 20 throughout the monitoring period. The hemodynamic monitor saves the second dataset 34 to the memory 14 of the hemodynamic monitor. In a first step the processor 12 populates a data sequence with all of the values of the first dataset 32 relative to time of the monitoring period.

The method of the preceding paragraph can optionally include, additionally and/or alternatively, any one or more of the following features, configurations and/or additional components in the paragraphs below.

In an embodiment of the foregoing method, the method further comprises: populating through a second step, by the processor 12, any segments of time missing a hemodynamic parameter value in the data sequence with a hemodynamic parameter value from a corresponding time segment in the second dataset 34 after completion of the first step; and outputting, by the processor 12, the data sequence to a display 16.

In an embodiment of the foregoing method, the method further comprises: sensing the hemodynamic parameter of the patient with a third sensor 22 and communicating a third dataset 36 of the hemodynamic parameter to the hemodynamic monitor by the third sensor 22 throughout the monitoring period of the patient 11; saving, by the hemodynamic monitor, the third dataset 36 to the memory of the hemodynamic monitor; assigning, by a processor 12, a third priority to the third dataset 36; populating through a third step, by the processor 12, any segments of time missing a hemodynamic parameter value in the data sequence with a hemodynamic parameter value from a corresponding time segment in the third dataset 36 after completion of the second step; and outputting, by the processor 12, the data sequence to a display 16.

In an embodiment of the foregoing method, the third sensor is an invasive hemodynamic sensor 20, a minimally invasive hemodynamic sensor 18, or a non-invasive hemodynamic sensor 22.

In an embodiment of the foregoing method, the first sensor is an invasive hemodynamic sensor 20, a minimally invasive hemodynamic sensor 18, or a non-invasive hemodynamic sensor 22.

In an embodiment of the foregoing method, the second sensor is an invasive hemodynamic sensor 20, a minimally invasive hemodynamic sensor 18, or a non-invasive hemodynamic sensor 22.

In another aspect of the disclosure, a method is provided for monitoring of a hemodynamic parameter of a patient 11. The method includes receiving, at a hemodynamic monitor, a first dataset 32 of a hemodynamic parameter of the patient 11 from a first sensor 18 throughout a monitoring period of the patient 11; saving, by the hemodynamic monitor, the first dataset 32 to a memory 14 of the hemodynamic monitor; receiving, at a hemodynamic monitor, a second dataset 34 of the hemodynamic parameter of the patient from a second sensor 20 throughout the monitoring period; and saving, by the hemodynamic monitor, the second dataset 34 to the memory 14 of the hemodynamic monitor. In a first step the processor 12 populates a data sequence with all of the values of the first dataset 32 relative to time of the monitoring period. This method can optionally include, *mutatis mutandis*, any of the features of the methods described in the preceding paragraphs.

Combinations of features from different specific implementations described above form part of the present disclosure. The methods described herein can be performed using the systems described herein, or a system comprising any one or more components of the systems described herein.

Methods disclosed herein also encompass analogous methods performed on a (non-living) simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (e.g., thorax), a system (e.g., cardiovascular system), an organ (e.g., heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loudspeakers, headphones, pressure transducers, temperature transducers, or using any combination of suitable technologies.

### Discussion of Possible Embodiments

The following are non-exclusive descriptions of possible embodiments of the present disclosure.

In one aspect of the disclosure, a method for monitoring of a hemodynamic parameter of a patient is disclosed. The method includes sensing the hemodynamic parameter of the patient with a first sensor. The first sensor communicates a first dataset of the hemodynamic parameter to a hemodynamic monitor throughout a monitoring period of the patient. The hemodynamic monitor saves the first dataset to a memory of the hemodynamic monitor. The method also includes sensing the hemodynamic parameter of the patient with a second sensor. The second sensor communicates a second dataset of the hemodynamic parameter to the hemodynamic monitor throughout the monitoring period. The hemodynamic monitor saves the second dataset to the memory of the hemodynamic monitor. A processor assigns a first priority to the first dataset. The processor assigns a second priority to the second dataset. In a first step, the processor populates a data sequence with all of the values of the first dataset relative to time of the monitoring period. In a second step, the processor populates any segments of time missing a hemodynamic parameter value in the data sequence with a hemodynamic parameter value from a corresponding time segment in the second dataset after completion of the first step. The processor outputs the data sequence to a display.

The method of the preceding paragraph can optionally include, additionally and/or alternatively, any one or more of the following features, configurations and/or additional components in the paragraphs below.

In an embodiment of the foregoing method, the first sensor is an invasive hemodynamic sensor, a minimally invasive hemodynamic sensor, or a non-invasive hemodynamic sensor; and wherein the second sensor is an invasive hemodynamic sensor, a minimally invasive hemodynamic sensor, or a non-invasive hemodynamic sensor.

In an embodiment of the foregoing method, the method further comprises: sensing the hemodynamic parameter of the patient with a third sensor and communicating a third dataset of the hemodynamic parameter to the hemodynamic monitor by the third sensor throughout the monitoring period of the patient; saving, by the hemodynamic monitor, the third dataset to the memory of the hemodynamic monitor; assigning, by a processor, a third priority to the third dataset; populating through a third step, by the processor, any segments of time missing a hemodynamic parameter value in the data sequence with a hemodynamic parameter value from a corresponding time segment in the third dataset after completion of the second step; and outputting, by the processor, the data sequence to a display.

In an embodiment of the foregoing method, the third sensor is an invasive hemodynamic sensor, a minimally invasive hemodynamic sensor, or a non-invasive hemodynamic sensor.

In an embodiment of the foregoing method, the processor is a computer processor of a device physically separate from the hemodynamic monitor.

In an embodiment of the foregoing method, the processor is a processor of the hemodynamic monitor.

In an embodiment of the foregoing method, the assigning by the processor the first priority to the first dataset is in response to a first input from a user through a user interface in communication with the processor; and wherein the assigning by the processor the second priority to the second dataset is in response to a second input from the user through the user interface.

In an embodiment of the foregoing method, the hemodynamic parameter is at least one of arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility of the patient.

In another aspect of the disclosure, a system for monitoring of a hemodynamic parameter of a patient includes a first hemodynamic sensor and a second hemodynamic sensor. The first hemodynamic sensor is configured to sense the hemodynamic parameter of the patient and produce a first dataset of the hemodynamic parameter of the patient. The second hemodynamic sensor is configured to sense the hemodynamic parameter of the patient and produce a second dataset of the hemodynamic parameter of the patient. A hemodynamic monitor is in communication with the first hemodynamic sensor and the second hemodynamic sensor. A system memory is configured to receive the first dataset and the second dataset from the hemodynamic monitor. The system memory also stores aggregating tool software code. The system also includes a hardware processor that is configured to execute the aggregating software code to assign a first priority to the first dataset, assign a second priority to the second dataset, and perform a first step by populating a data sequence with all of the values of the first dataset relative to time of a monitoring period. The hardware processor is also configured to execute the aggregating software code to perform a second step by populating any segments of time missing a hemodynamic parameter value in the data sequence with a hemodynamic parameter value from a corresponding time segment in the second dataset after completion of the first step. The hardware processor is also configured to execute the aggregating software code to output the data sequence after completion of the second step to a display in communication with the processor.

The system of the preceding paragraph can optionally include, additionally and/or alternatively, any one or more of the following features, configurations and/or additional components in the paragraphs below.

In an embodiment of the foregoing system, the first hemodynamic sensor is a non-invasive hemodynamic sensor that is attachable to an extremity of the patient, wherein the first hemodynamic sensor is a minimally invasive hemodynamic sensor connected to a catheter that is inserted into an extremity of the patient, or wherein the first hemodynamic pressure sensor is an invasive hemodynamic pressure sensor connected to a cardiac catheter that is inserted into a heart of the patient.

In an embodiment of the foregoing system, the second hemodynamic sensor is a non-invasive hemodynamic sensor that is attachable to an extremity of the patient, wherein the second hemodynamic sensor is a minimally invasive hemodynamic sensor connected to a catheter that is inserted into an extremity of the patient, or wherein the second hemodynamic pressure sensor is an invasive hemodynamic pressure sensor connected to a cardiac catheter that is inserted into a heart of the patient.

In an embodiment of the foregoing system, the system further comprises: a third hemodynamic sensor in communication with the hemodynamic monitor and configured to sense the hemodynamic parameter of the patient and produce a third dataset of the hemodynamic parameter of the patient, wherein the system memory configured to receive the third dataset from the hemodynamic monitor, and wherein the hardware processor is configured to execute the aggregating software code to: assign a third priority to the third dataset; perform a third step by populating any segments of time missing a hemodynamic parameter value in the data sequence with a hemodynamic parameter value from a corresponding time segment in the third dataset after completion of the second step; and outputting the data sequence after completion of the third step to the display.

In an embodiment of the foregoing system, the third hemodynamic sensor is a non-invasive hemodynamic sensor that is attachable to an extremity of the patient, wherein the third hemodynamic sensor is a minimally invasive hemodynamic sensor connected to a catheter that is inserted into an extremity of the patient, or wherein the third hemodynamic pressure sensor is an invasive hemodynamic pressure sensor connected to a cardiac catheter that is inserted into a heart of the patient.

In an embodiment of the foregoing system, the system further comprises a computer system separate from the hemodynamic monitor and comprising the system memory and the hardware processor.

In another aspect of the disclosure, a method is disclosed for monitoring of a hemodynamic parameter of a patient. The method includes sensing the hemodynamic parameter of the patient with a first sensor and communicating a first dataset of the hemodynamic parameter to a hemodynamic monitor by the first sensor throughout a monitoring period of the patient. The hemodynamic monitor saves the first dataset to a memory of the hemodynamic monitor. The method also includes sensing the hemodynamic parameter of the patient with a second sensor and communicating a second dataset of the hemodynamic parameter to the hemodynamic monitor by the second sensor throughout the monitoring period. The hemodynamic monitor saves the second dataset to the memory of the hemodynamic monitor. In a first step the processor populates a data sequence with all of the values of the first dataset relative to time of the monitoring period.

The method of the preceding paragraph can optionally include, additionally and/or alternatively, any one or more of the following features, configurations and/or additional components in the paragraphs below.

In an embodiment of the foregoing method, the method further comprises: populating through a second step, by the processor, any segments of time missing a hemodynamic parameter value in the data sequence with a hemodynamic parameter value from a corresponding time segment in the second dataset after completion of the first step; and outputting, by the processor, the data sequence to a display.

In an embodiment of the foregoing method, the method further comprises: sensing the hemodynamic parameter of the patient with a third sensor and communicating a third dataset of the hemodynamic parameter to the hemodynamic monitor by the third sensor throughout the monitoring period of the patient; saving, by the hemodynamic monitor, the third dataset to the memory of the hemodynamic monitor; assigning, by a processor, a third priority to the third dataset; populating through a third step, by the processor, any segments of time missing a hemodynamic parameter value in the data sequence with a hemodynamic parameter value from a corresponding time segment in the third dataset after completion of the second step; and outputting, by the processor, the data sequence to a display.

In an embodiment of the foregoing method, the third sensor is an invasive hemodynamic sensor, a minimally invasive hemodynamic sensor, or a non-invasive hemodynamic sensor.

In an embodiment of the foregoing method, the first sensor is an invasive hemodynamic sensor, a minimally invasive hemodynamic sensor, or a non-invasive hemodynamic sensor.

In an embodiment of the foregoing method, the second sensor is an invasive hemodynamic sensor, a minimally invasive hemodynamic sensor, or a non-invasive hemodynamic sensor.

The disclosure comprises the following items:
1. A method for monitoring of a hemodynamic parameter of a patient, the method comprising:
   sensing the hemodynamic parameter of the patient with a first sensor and communicating a first dataset of the hemodynamic parameter to a hemodynamic monitor by the first sensor throughout a monitoring period of the patient;
   saving, by the hemodynamic monitor, the first dataset to a memory of the hemodynamic monitor;
   sensing the hemodynamic parameter of the patient with a second sensor and communicating a second dataset of the hemodynamic parameter to the hemodynamic monitor by the second sensor throughout the monitoring period;
   saving, by the hemodynamic monitor, the second dataset to the memory of the hemodynamic monitor;
   assigning, by a processor, a first priority to the first dataset;
   assigning, by the processor, a second priority to the second dataset;
   populating through a first step, by the processor, a data sequence with all of the values of the first dataset relative to time of the monitoring period;
   populating through a second step, by the processor, any segments of time missing a hemodynamic parameter value in the data sequence with a hemodynamic parameter value from a corresponding time segment in the second dataset after completion of the first step; and
   outputting, by the processor, the data sequence to a display.
2. The method of item 1, further comprising:
   sensing the hemodynamic parameter of the patient with a third sensor and communicating a third dataset of the hemodynamic parameter to the hemodynamic monitor by the third sensor throughout the monitoring period of the patient;
   saving, by the hemodynamic monitor, the third dataset to the memory of the hemodynamic monitor;
   assigning, by a processor, a third priority to the third dataset;
   populating through a third step, by the processor, any segments of time missing a hemodynamic parameter value in the data sequence with a hemodynamic parameter value from a corresponding time segment in the third dataset after completion of the second step; and
   outputting, by the processor, the data sequence to a display.
3. The method of items 1 or 2, wherein the first sensor is an invasive hemodynamic sensor, a minimally invasive hemodynamic sensor, or a non-invasive hemodynamic sensor.
4. The method of any preceding item, wherein the second sensor is an invasive hemodynamic sensor, a minimally invasive hemodynamic sensor, or a non-invasive hemodynamic sensor.
5. The method of any preceding item, wherein the third sensor is an invasive hemodynamic sensor, a minimally invasive hemodynamic sensor, or a non-invasive hemodynamic sensor.
6. The method of any preceding item, wherein the processor is a computer processor of a device physically separate from the hemodynamic monitor.
7. The method of any of items 1-5, wherein the processor is a processor of the hemodynamic monitor.
8. The method of any preceding item, wherein the assigning by the processor the first priority to the first dataset is in response to a first input from a user through a user interface in communication with the processor.
9. The method of item 8, wherein the assigning by the processor the second priority to the second dataset is in response to a second input from the user through the user interface.
10. A method for monitoring a hemodynamic parameter of a patient, the method comprising:
   sensing the hemodynamic parameter of the patient with a first sensor and communicating a first dataset of the hemodynamic parameter to a hemodynamic monitor by the first sensor throughout a monitoring period of the patient;
   saving, by the hemodynamic monitor, the first dataset to a memory of the hemodynamic monitor;
   sensing the hemodynamic parameter of the patient with a second sensor and communicating a second dataset of the hemodynamic parameter to the hemodynamic monitor by the second sensor throughout the monitoring period;
   saving, by the hemodynamic monitor, the second dataset to the memory of the hemodynamic monitor;
   populating through a first step, by a processor, a data sequence with all of the values of the first dataset relative to time of the monitoring period;
   populating through a second step, by the processor, any segments of time missing a hemodynamic parameter value in the data sequence with a hemodynamic parameter value from a corresponding time segment in the second dataset after completion of the first step; and
   outputting, by the processor, the data sequence to a display.
11. The method of item 10, wherein the hemodynamic parameter is at least one of arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility of the patient.
12. The method of items 10 or 11, wherein the first sensor is an invasive hemodynamic sensor with a cardiac catheter, a minimally invasive hemodynamic sensor, or a non-invasive hemodynamic sensor.
13. The method of any of items 10-12, wherein the second sensor is an invasive hemodynamic sensor with a cardiac catheter, a minimally invasive hemodynamic sensor, or a non-invasive hemodynamic sensor.
14. A system for monitoring of a hemodynamic parameter of a patient, the system comprising:
   a first hemodynamic sensor configured to sense the hemodynamic parameter of the patient and produce a first dataset of the hemodynamic parameter of the patient;
   a second hemodynamic sensor configured to sense the hemodynamic parameter of the patient and produce a second dataset of the hemodynamic parameter of the patient;
   a hemodynamic monitor in communication with the first hemodynamic sensor and the second hemodynamic sensor;
   a system memory configured to receive the first dataset and the second dataset from the hemodynamic monitor, wherein the system memory stores aggregating tool software code;
   a hardware processor that is configured to execute the aggregating software code to:
      assign a first priority to the first dataset;
      assign a second priority to the second dataset;
      perform a first step by populating a data sequence with all of the values of the first dataset relative to time of a monitoring period;
      perform a second step by populating any segments of time missing a hemodynamic parameter value in the data sequence with a hemodynamic parameter value from a corresponding time segment in the second dataset after completion of the first step; and
      outputting the data sequence after completion of the second step to a display in communication with the processor.
15. The system of item 14, wherein the first hemodynamic sensor is a non-invasive hemodynamic sensor that is attachable to an extremity of the patient, wherein the first hemodynamic sensor is a minimally invasive hemodynamic sensor connected to a catheter that is inserted into an extremity of the patient, or wherein the first hemodynamic pressure sensor is an invasive hemodynamic pressure sensor connected to a cardiac catheter that is inserted into a heart of the patient.
16. The system of item 15, wherein the second hemodynamic sensor is a non-invasive hemodynamic sensor that is attachable to an extremity of the patient, wherein the second hemodynamic sensor is a minimally invasive hemodynamic sensor connected to a catheter that is inserted into an extremity of the patient, or wherein the second hemodynamic pressure sensor is an invasive hemodynamic pressure sensor connected to a cardiac catheter that is inserted into a heart of the patient.
17. The system of any of items 14-16, wherein the system further comprises:
   a third hemodynamic sensor in communication with the hemodynamic monitor and configured to sense the hemodynamic parameter of the patient and produce a third dataset of the hemodynamic parameter of the patient,
   wherein the system memory configured to receive the third dataset from the hemodynamic monitor, and
   wherein the hardware processor is configured to execute the aggregating software code to:
      assign a third priority to the third dataset;
      perform a third step by populating any segments of time missing a hemodynamic parameter value in the data sequence with a hemodynamic parameter value from a corresponding time segment in the third dataset after completion of the second step; and
      outputting the data sequence after completion of the third step to the display.
18. The system of item 17, wherein the third hemodynamic sensor is a non-invasive hemodynamic sensor that is attachable to an extremity of the patient, wherein the third hemodynamic sensor is a minimally invasive hemodynamic sensor connected to a catheter that is inserted into an extremity of the patient, or wherein the third hemodynamic pressure sensor is an invasive hemodynamic pressure sensor connected to a cardiac catheter that is inserted into a heart of the patient.
19. The system of any of items 14-18, further comprising:
   a computer system separate from the hemodynamic monitor and comprising the system memory and the hardware processor.
20. A method for monitoring of a hemodynamic parameter of a patient, the method comprising:
   sensing the hemodynamic parameter of the patient with a first sensor and communicating a first dataset of the hemodynamic parameter to a hemodynamic monitor by the first sensor throughout a monitoring period of the patient;
   saving, by the hemodynamic monitor, the first dataset to a memory of the hemodynamic monitor;
   sensing the hemodynamic parameter of the patient with a second sensor and communicating a second dataset of the hemodynamic parameter to the hemodynamic monitor by the second sensor throughout the monitoring period;
   saving, by the hemodynamic monitor, the second dataset to the memory of the hemodynamic monitor;
   assigning, by a processor, a first priority to the first dataset;
   assigning, by the processor, a second priority to the second dataset; and
   populating through a first step, by the processor, a data sequence with all of the values of the first dataset relative to time of the monitoring period.
21. The method of item 21, further comprising:
   populating through a second step, by the processor, any segments of time missing a hemodynamic parameter value in the data sequence with a hemodynamic parameter value from a corresponding time segment in the second dataset after completion of the first step; and
   outputting, by the processor, the data sequence to a display.
22. The method of item 21, further comprising:
   sensing the hemodynamic parameter of the patient with a third sensor and communicating a third dataset of the hemodynamic parameter to the hemodynamic monitor by the third sensor throughout the monitoring period of the patient;
   saving, by the hemodynamic monitor, the third dataset to the memory of the hemodynamic monitor;
   assigning, by a processor, a third priority to the third dataset;
   populating through a third step, by the processor, any segments of time missing a hemodynamic parameter value in the data sequence with a hemodynamic parameter value from a corresponding time segment in the third dataset after completion of the second step; and
   outputting, by the processor, the data sequence to a display.
23. The method of item 22, wherein the third sensor is an invasive hemodynamic sensor, a minimally invasive hemodynamic sensor, or a non-invasive hemodynamic sensor.
24. The method of any of items 20-23, wherein the first sensor is an invasive hemodynamic sensor, a minimally invasive hemodynamic sensor, or a non-invasive hemodynamic sensor.
25. The method of any of items 20-24, wherein the second sensor is an invasive hemodynamic sensor, a minimally invasive hemodynamic sensor, or a non-invasive hemodynamic sensor.
26. The method of any of items 20-25, wherein the processor is a computer processor of a device physically separate from the hemodynamic monitor.
27. The method of any of items 20-26, wherein the processor is a processor of the hemodynamic monitor.
28. The method of any of items 20-27, wherein the assigning by the processor the first priority to the first dataset is in response to a first input from a user through a user interface in communication with the processor.
29. The method of item 28, wherein the assigning by the processor the second priority to the second dataset is in response to a second input from the user through the user interface.

While the disclosure has been described with reference to an exemplary embodiment(s), it will be understood by those skilled in the art that various changes may be made and equivalents may be substituted for elements thereof without departing from the scope of the disclosure. In addition, many modifications may be made to adapt a particular situation or material to the teachings of the disclosure without departing from the essential scope thereof. Therefore, it is intended that the disclosure not be limited to the particular embodiment(s) disclosed, but that the disclosure will include all embodiments falling within the scope of the appended claims.

## Claims

1. A method for monitoring of a hemodynamic parameter of a patient, the method comprising:
sensing the hemodynamic parameter of the patient with a first sensor and communicating a first dataset of the hemodynamic parameter to a hemodynamic monitor by the first sensor throughout a monitoring period of the patient;
saving, by the hemodynamic monitor, the first dataset to a memory of the hemodynamic monitor;
sensing the hemodynamic parameter of the patient with a second sensor and communicating a second dataset of the hemodynamic parameter to the hemodynamic monitor by the second sensor throughout the monitoring period;
saving, by the hemodynamic monitor, the second dataset to the memory of the hemodynamic monitor;
assigning, by a processor, a first priority to the first dataset;
assigning, by the processor, a second priority to the second dataset;
populating through a first step, by the processor, a data sequence with all of the values of the first dataset relative to time of the monitoring period;
populating through a second step, by the processor, any segments of time missing a hemodynamic parameter value in the data sequence with a hemodynamic parameter value from a corresponding time segment in the second dataset after completion of the first step; and
outputting, by the processor, the data sequence to a display.

2. The method of claim 1, wherein the first sensor is an invasive hemodynamic sensor, a minimally invasive hemodynamic sensor, or a non-invasive hemodynamic sensor; and/or wherein the second sensor is an invasive hemodynamic sensor, a minimally invasive hemodynamic sensor, or a non-invasive hemodynamic sensor.

3. The method of claim 1 or claim 2, further comprising:
sensing the hemodynamic parameter of the patient with a third sensor and communicating a third dataset of the hemodynamic parameter to the hemodynamic monitor by the third sensor throughout the monitoring period of the patient;
saving, by the hemodynamic monitor, the third dataset to the memory of the hemodynamic monitor;
assigning, by a processor, a third priority to the third dataset;
populating through a third step, by the processor, any segments of time missing a hemodynamic parameter value in the data sequence with a hemodynamic parameter value from a corresponding time segment in the third dataset after completion of the second step; and
outputting, by the processor, the data sequence to a display.

4. The method of claim 3, wherein the third sensor is an invasive hemodynamic sensor, a minimally invasive hemodynamic sensor, or a non-invasive hemodynamic sensor.

5. The method of any preceding claim, wherein the processor is a computer processor of a device physically separate from the hemodynamic monitor.

6. The method of any of claims 1 to 5, wherein the processor is a processor of the hemodynamic monitor.

7. The method of any preceding claim, wherein the assigning by the processor the first priority to the first dataset is in response to a first input from a user through a user interface in communication with the processor; and/or wherein the assigning by the processor the second priority to the second dataset is in response to a second input from the user through the user interface.

8. The method of any preceding claim, wherein the hemodynamic parameter is at least one of arterial pressure, cardiac output, stroke volume, stroke volume variation, heart rate, respiratory rate, pulse pressure, pulse pressure variation, mean arterial pressure (MAP), systolic pressure (SYS), diastolic pressure (DIA), heart rate variability, peripheral resistance, vascular compliance, dynamic arterial elastance, and/or left-ventricular contractility of the patient.

9. A system for monitoring of a hemodynamic parameter of a patient, the system comprising:
a first hemodynamic sensor configured to sense the hemodynamic parameter of the patient and produce a first dataset of the hemodynamic parameter of the patient;
a second hemodynamic sensor configured to sense the hemodynamic parameter of the patient and produce a second dataset of the hemodynamic parameter of the patient;
a hemodynamic monitor in communication with the first hemodynamic sensor and the second hemodynamic sensor;
a system memory configured to receive the first dataset and the second dataset from the hemodynamic monitor, wherein the system memory stores aggregating tool software code;
a hardware processor that is configured to execute the aggregating software code to:
assign a first priority to the first dataset;
assign a second priority to the second dataset;
perform a first step by populating a data sequence with all of the values of the first dataset relative to time of a monitoring period;
perform a second step by populating any segments of time missing a hemodynamic parameter value in the data sequence with a hemodynamic parameter value from a corresponding time segment in the second dataset after completion of the first step; and
outputting the data sequence after completion of the second step to a display in communication with the processor.

10. The system of claim 9, wherein the first hemodynamic sensor is a non-invasive hemodynamic sensor that is attachable to an extremity of the patient, wherein the first hemodynamic sensor is a minimally invasive hemodynamic sensor connected to a catheter that is inserted into an extremity of the patient, or wherein the first hemodynamic pressure sensor is an invasive hemodynamic pressure sensor connected to a cardiac catheter that is inserted into a heart of the patient.

11. The system of claim 9 or claim 10, wherein the second hemodynamic sensor is a non-invasive hemodynamic sensor that is attachable to an extremity of the patient, wherein the second hemodynamic sensor is a minimally invasive hemodynamic sensor connected to a catheter that is inserted into an extremity of the patient, or wherein the second hemodynamic pressure sensor is an invasive hemodynamic pressure sensor connected to a cardiac catheter that is inserted into a heart of the patient.

12. The system of any of claims 9 to 11, wherein the system further comprises:
a third hemodynamic sensor in communication with the hemodynamic monitor and configured to sense the hemodynamic parameter of the patient and produce a third dataset of the hemodynamic parameter of the patient,
wherein the system memory configured to receive the third dataset from the hemodynamic monitor, and
wherein the hardware processor is configured to execute the aggregating software code to:
assign a third priority to the third dataset;
perform a third step by populating any segments of time missing a hemodynamic parameter value in the data sequence with a hemodynamic parameter value from a corresponding time segment in the third dataset after completion of the second step; and
outputting the data sequence after completion of the third step to the display.

13. The system of claim 12, wherein the third hemodynamic sensor is a non-invasive hemodynamic sensor that is attachable to an extremity of the patient, wherein the third hemodynamic sensor is a minimally invasive hemodynamic sensor connected to a catheter that is inserted into an extremity of the patient, or wherein the third hemodynamic pressure sensor is an invasive hemodynamic pressure sensor connected to a cardiac catheter that is inserted into a heart of the patient.

14. The system of any of claims 9 to 13, further comprising:
a computer system separate from the hemodynamic monitor and comprising the system memory and the hardware processor.
